# EUROPEAN PATENT SPECIFICATION

(11) **EP 2 061 501 B1**
(45) Date of publication and mention of the grant of the patent: **08.04.2015**
(21) Application number: 07804202.5
(22) Date of filing: 10.09.2007
(51) Int. Cl.: A61K 38/57, A61P 11/06, A61P 11/00, A61K 38/16

(54) **METHOD OF TREATING RESPIRATORY DISORDERS**
VERFAHREN ZUR BEHANDLUNG VON ATEMWEGSERKRANKUNGEN
PROCÉDÉ DE TRAITEMENT DE TROUBLES RESPIRATOIRES

(30) Priority: 08.09.2006 GB 0617735; 22.11.2006 US 860730 P
(43) Date of publication of application: 27.05.2009
(73) Proprietor: Volution Immuno Pharmaceuticals SA, 3461-1211 Geneve 3 (CH)
(72) Inventor: HAMER, John, London W1T 4EU (GB)
(74) Representative: Goodfellow, Hugh Robin
(86) International application number: PCT/GB2007/003404
(87) International publication number: WO 2008/029169

(56) References cited:
- WO-A-2004/106369
- US-A1- 2004 115 194

## Description

The present invention relates to the use of agents that bind the complement protein C5 in the treatment of diseases associated with inappropriate complement activation, and in particular in the treatment of respiratory disorders.

### Background to the invention

The complement system is an essential part of the body's natural defence mechanism against foreign invasion and is also involved in the inflammatory process. More than 30 proteins in serum and at the cell surface are involved in complement system function and regulation. Recently it has become apparent that, as well as the ∼35 known components of the complement system which may be associated with both beneficial and pathological processes, the complement system itself interacts with at least 85 biological pathways with functions as diverse as angiogenesis, platelet activation, glucose metabolism and spermatogenesis [1].

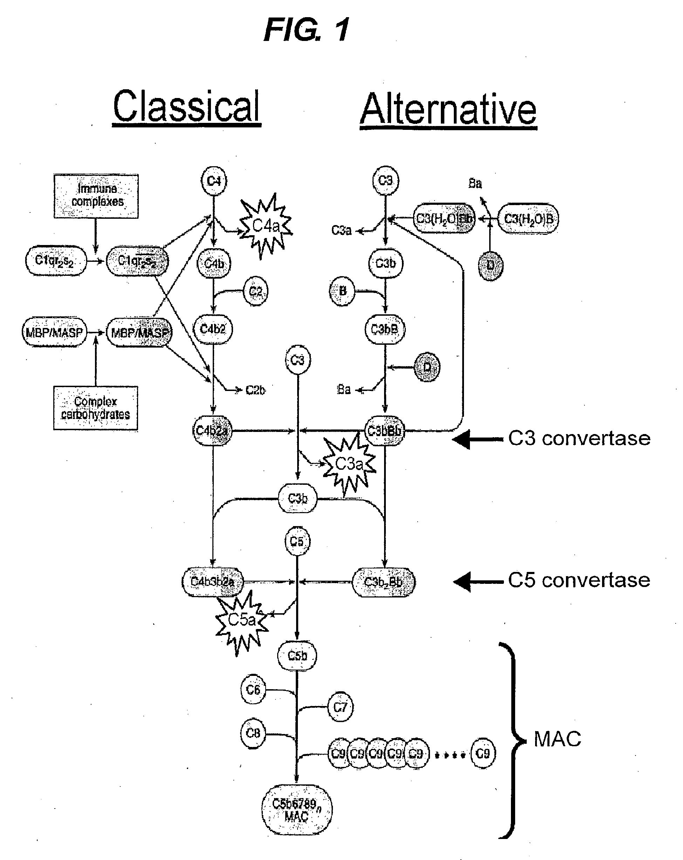

The complement system is activated by the presence of foreign antigens. Three activation pathways exist: (1) the classical pathway which is activated by IgM and IgG complexes or by recognition of carbohydrates; (2) the alternative pathway which is activated by non-self surfaces (lacking specific regulatory molecules) and by bacterial endotoxins; and (3) the lectin pathway which is activated by binding of manna-binding lectin (MBL) to mannose residues on the surface of a pathogen. The three pathways comprise parallel cascades of events that result in the production of complement activation through the formation of similar C3 and C5 convertases on cell surfaces resulting in the release of acute mediators of inflammation (C3a and C5a) and formation of the membrane attack complex (MAC). The parallel cascades involved in the classical and alternative pathways are shown in Figure 1.

Complement can be activated inappropriately under certain circumstances leading to undesirable local tissue destruction. Inappropriate complement activation has been shown to play a role in a wide variety of diseases and disorders including acute pancreatitis, Alzheimer's disease, allergic encephalomyelitis, allotransplatation, asthma, adult respiratory distress syndrome, burn injuries, Crohn's disease, glomerulonephritis, haemolytic anaemia, haemodialysis, hereditary angioedema, ischaemia reperfusion injuries, multiple system organ failure, multiple sclerosis, myasthenia gravis, ischemic stroke, myocardial infarction, psoriasis, rheumatoid arthritis, septic shock, systemic lupus erythematosus, stroke, vascular leak syndrome, transplantation rejection and inappropriate immune response in cardiopulmonary bypass operations. Inappropriate activation of the complement system has thus been a target for therapeutic intervention for many years and numerous complement inhibitors targeting different parts of the complement cascade are under development for therapeutic use.

In ischemic stroke and myocardial infarction, the body recognises the dead tissue in the brain or heart as foreign and activates complement so causing further local damage. Similarly in cardiopulmonary bypass operations, the body recognises the plastic surfaces in the machine as foreign, activates complement and can result in vascular damage. In autoimmune diseases, the body may wrongly recognise itself as foreign and activate complement with local tissue damage (e.g. joint destruction in rheumatoid arthritis and muscle weakness in myasthenia gravis).

Several experimental models for bronchial asthma have indicated that C5 and its activated components are involved in the development of airway inflammation and bronchoconstriction. Studies with various complement inhibitors markedly reduced airway hyperresponsiveness or airway inflammation in rodents [2,3,4].

COPD is a general term, which includes the conditions chronic bronchitis and emphysema and is used to describe airways that are narrowed due to chronic bronchitis, emphysema or both. COPD mainly affects people over the age of 40 and smoking is the cause in the majority of cases. The symptoms include a persistent cough, breathlessness and wheeze resulting in an increasing difficulty with breathing.

Asthma is characterised by a combination of chronic airway inflammation, airway obstruction, and airway hyperresponsiveness to various stimuli. It is thought to be mediated primarily by adaptive immune responses mediated by allergen-specific CD4+ T cells; Th2 cytokines and allergen specific IgE, which lead to pulmonary inflammation and airway hyperresponsiveness.

In COPD there is permanent damage to the airways. The narrowed airways are 'fixed' and so symptoms are chronic. Treatment to open up the airways is limited. In asthma, there is inflammation in the airways which causes muscles in the airways to constrict. This causes the airways to narrow. The symptoms tend to come and go and vary in severity from time to time. Treatment to reduce inflammation and to open up the airways usually works well. Both asthma and COPD are presently treated using varying combinations of bronchodilators, for example beta agonists such as salbutamol and terbutaline and anticholinergic agents such as ipratropium and oxitropium; and steroids such as beclomethasone, budesonide and fluticasone. However, further improved treatments would be beneficial.

There is a great need for agents that improve upon the currently available treatments for respiratory disorders such as COPD and asthma.

### Summary of the invention:

Accordingly, the invention provides a method of treating or preventing a respiratory disorder comprising administering to a subject in need thereof a therapeutically or prophylactically effective amount of an agent that binds complement C5.

The invention also provides the use of a therapeutically or prophylactically effective amount of an agent that binds complement C5 in the manufacture of a medicament for treating or preventing a respiratory disorder.

The respiratory disorder of the invention includes one or more of asthma, including severe and steroid resistant asthma, COPD, immune complex alveolitis including those caused by exposure to organic dusts, moulds, airborne allergens, mineral dust, chemicals etc. Such conditions include but are not limited to: farmer's lung, pigeon or bird fancier's lung, barn fever, miller's lung, metalworker's lung, humidifier fever, silicosis, pneumoconiosis, asbestosis, byssinosis, berylliosis, mesothelioma. Asthma, rhinitis, alveolitis or diffuse fibrotic lung disease caused by exposure to systemic or inhaled drugs and chemical agents including but not limited to: bleomycin, mitomycin, penicillins, sulphonamides, cephalosporins, aspirin, NSAIDs, tartrazine, ACE inhibitors, iodine containing contrast media, non-selective β blocking drugs, suxamethonium, hexamethonium, thiopentone, amiodarone, nitrofurantoin, paraquat, oxygen, cytotoxic agents, tetracyclines, phenytoin, carbamazepine, chlorpropamide, hydralazine, procainamide, isoniazid, *p*-aminosalicylic acid. Physical lung damage including but not limited to: crush injury, smoke and hot gas inhalation, blast injury, radiation injury. aspiration pneumonitis. lipoid pneumonia. Lung damage associated with organ transplantation including but not limited to: cardiac transplantation, lung transplantation, bone marrow transplantation. Cryptogenic fibrosing alveolitis. Allergic granulomatosis (Churg-Strauss syndrome). Wegener's granulomatosis. Broncheolitis obliterans. Interstitial pulmonary fibrosis. Cystic fibrosis. Respiratory manifestations of autoimmune and connective tissue diseases including but not limited to: rheumatoid disease, systemic lupus erythematosus, systemic sclerosis, polyarteritis nodosa, polymyositis, dermatomyositis, sjögren's syndrome, ankylosing spondylitis, caplan's syndrome. Goodpasture's syndrome. Pulmonary alveolar proteinosis. Idiopathic pulmonary haemosiderosis. Histiocytosis X. Pulmonary infiltration with eosinophilia (PIE) including but not limited to: simple pulmonary eosinophilia, prolonged pulmonary eosinophilia, asthmatic bronchopulmonary eosinophilia, allergic bronchopulmonary aspergillosis, aspergilloma, invasive aspergillosis, tropical pulmonary eosinophilia, hypereosinohilic syndrome, parasitic infestation. Lymphangioleiomyomatosis (LAM).

Preferably, the respiratory disorder is either COPD or asthma.

Preferably, the agent acts to prevent the cleavage of complement C5 by C5 convertase into complement C5a and complement C5b-9.

The complement C5 protein, also referred to herein as C5, is cleaved by the C5 convertase enzyme, itself formed from C3a, an earlier product of the alternative pathway (Figure 1). The products of this cleavage include an anaphylatoxin C5a and a lytic complex C5b - 9 also known as membrane attack complex (MAC). C5a is a highly reactive peptide implicated in many pathological inflammatory processes including neutrophil and eosinophil chemotaxis, neutrophil activation, increased capillary permeability and inhibition of neutrophil apoptosis [5].

MAC is associated with other important pathological processes including rheumatoid arthritis [6;7], proliferative glomerulonephritis [8], idiopathic membranous nephropathy [9], proteinurea [10], demyelination after acute axonal injury [11] and is also responsible for acute graft rejection following xenotransplantation [12].

C5a has become a target of particular interest in the field of complement-associated disorders [13]. Although C5a has many well-recognised pathological associations, the effects of its depletion in humans appear to be limited. Monoclonal antibodies and small molecules that bind and inhibit C5a or C5a receptors have been developed to treat various autoimmune diseases. These molecules do not, however, prevent the release of MAC.

In contrast, administration of an agent that binds C5 according to the first aspect of the invention, inhibits both the formation of C5a peptide and the MAC. Surprisingly, it has been found that inhibition of both C5a and the MAC reduces the clinical symptoms associated with respiratory disorders. Furthermore, because C5 is a late product of the classical and alternative complement pathways, inhibition of C5 is less likely to be associated with risks of concomitant infection that exist when targeting earlier products in the cascade [14].

The ability of an agent to bind C5 may be determined by standard *in vitro* assays known in the art, for example by western blotting following incubation of the protein on the gel with labelled C5. Preferably, the agent according to the invention binds C5 with an IC₅₀ of less than 0.2 mg/ml, preferably less than 0.1 mg/ml, preferably less than 0.05 mg/ml, preferably less than 0.04 mg/ml, preferably less than 0.03 mg/ml, preferably 0.02 mg/ml, preferably less than 1µg/ml, preferably less than 100ng/ml, preferably less than 10ng/ml, more preferably still, less than 1ng/ml.

According to the invention, the agent that binds C5 is not an anti-C5 monoclonal antibody.

The agent that binds C5 is derived from a haematophagous arthropod. The term "haematophagous arthropod" includes all arthropods that take a blood meal from a suitable host, such as insects, ticks, lice, fleas and mites. Preferably, the agent is derived from a tick, preferably from the tick *Ornithodoros moubata.*

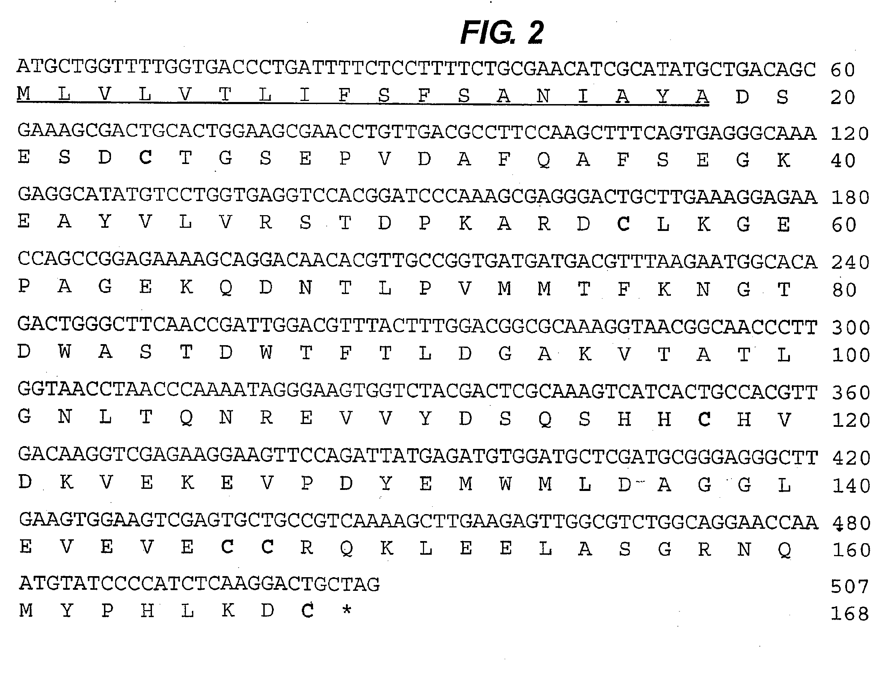

According to the invention, the agent that binds C5 is a protein comprising amino acids 19 to 168 of the amino acid sequence in Figure 2 or is a functional equivalent of this protein. The agent that binds C5 may be a protein consisting of amino acids 19 to 168 of the amino acid sequence in Figure 2 or be a functional equivalent of this protein.

According to an alternative embodiment, the protein used according to this embodiment of the invention may comprise or consist of amino acids 1 to 168 of the amino acid sequence in Figure 2, or be a functional equivalent thereof. The first 18 amino acids of the protein sequence given in Figure 2 form a signal sequence which is not required for C5 binding activity and so this may optionally be dispensed with, for example, for efficiency of recombinant protein production.

The protein having the amino acid sequence given in Figure 2, also referred to herein as the EV576 protein, was isolated from the salivary glands of the tick *Ornithodoros moubata.* EV576 is an outlying member of the lipocalin family and is the first lipocalin family member shown to inhibit complement activation. The EV576 protein inhibits the alternative, classical and lectin complement pathways by binding C5 and preventing its cleavage by C5 convertase into Complement C5a and Complement C5b - 9, thus inhibiting both the action of C5a peptide and the MAC. The term "EV576 protein", as used herein, refers to the sequence given in Figure 2 with or without the signal sequence.

The EV576 protein and the ability of this protein to inhibit complement activation has been disclosed in [20], where the EV576 protein was referred to as the "OmCI protein". It has now been found that the EV576 protein is surprisingly effective in the treatment and prevention of respiratory disorders. The data presented herein demonstrate that EV576 is potent in modulating acute asthma in the OVA asthma model. It reduces airway hyperresponsiveness to background levels at all doses tested, and reduces cellular infiltration into the lung. EV576 thus represents a potential human therapy for the treatment and prevention of respiratory disorders.

The surprising effectiveness of EV576 in the treatment of respiratory disorders appears to be due to the fact that it acts by binding C5, thus inhibiting the formation of C5a and MAC.

According to a further embodiment of the invention, the agent may be a nucleic acid molecule encoding the EV576 protein or a functional equivalent thereof. For example, gene therapy may be employed to effect the endogenous production of the EV576 protein by the relevant cells in the subject, either *in vivo* or *ex vivo.* Another approach is the administration of "naked DNA" in which the therapeutic gene is directly injected into the bloodstream or into muscle tissue.

Preferably, such a nucleic acid molecule comprises or consists of bases 53 to 507 of the nucleotide sequence in Figure 2. This nucleotide sequence encodes the EV576 protein in Figure 2 without the signal sequence. The first 54 bases of the nucleotide sequence in Figure 2 encode the signal sequence of which is not required for complement inhibitory activity. Alternatively, the nucleic acid molecule may comprise or consist of bases 1 to 507 of the nucleic acid sequence in Figure 2, which encodes the protein with the signal sequence.

The EV576 protein has been demonstrated to bind to C5 and prevent its cleavage by C5 convertase in rat, mouse and human serum with an IC₅₀ of approximately 0.02mg/ml. Preferably, functional equivalents of the EV576 protein which retain the ability to bind C5 with an IC₅₀ of less than 0.2 mg/ml, preferably less than 0.1 mg/ml, preferably less than 0.05 mg/ml, preferably less than 0.02 mg/ml, preferably less than 1µg/ml, preferably less than 100ng/ml, preferably less than 10ng/ml, more preferably still, less than 1ng/ml.

In one respect, the term "functional equivalent" is used herein to describe homologues and fragments of the EV576 protein which retain its ability to bind C5, and to prevent the cleavage of complement C5 by C5 convertase into complement C5a and complement C5b-9. The term "functional equivalent" also refers to molecules that are structurally similar to the EV576 protein or that contain similar or identical tertiary structure, particularly in the environment of the active site or active sites of the EV576 protein that binds to C5, such as synthetic molecules.

The term "homologue" is meant to include reference to paralogues and orthologues of the EV576 sequence that is explicitly identified in Figure 2, including, for example, the EV576 protein sequence from other tick species, including *Rhipicephalus appendiculatus*, *R. sanguineous, R. bursa, A. americanum, A. cajennense*, *A. hebraeum, Boophilus microplus*, *B. annulatus*, *B. decoloratus, Dermacentor reticulatus*, *D. andersoni, D. marginatus*, *D. variabilis, Haemaphysalis inermis*, *Ha. leachii, Ha. punctata, Hyalomma anatolicum anatolicum*, *Hy. dromedarii*, *Hy. marginatum marginatum*, *Ixodes ricinus, I. persulcatus*, *I. scapularis*, *I. hexagonus, Argas persicus*, *A. reflexus, Ornithodoros erraticus, O. moubata moubata, O. m. porcinus*, *and O. savignyi.* The term "homologue" is also meant to include the equivalent EV576 protein sequence from mosquito species, including those of the *Culex, Anopheles* and *Aedes* genera, particularly *Culex quinquefasciatus*, *Aedes aegypti* and *Anopheles gambiae;* flea species, such as *Ctenocephalides felis* (the cat flea); horseflies; sandflies; blackflies; tsetse flies; lice; mites; leeches; and flatworms. The native EV576 protein is thought to exist in *O*. *moubata* in another three forms of around 18kDa and the term "homologue" is meant to include these alternative forms of EV576.

Methods for the identification of homologues of the EV576 sequence given in Figure 2 will be clear to those of skill in the art. For example, homologues may be identified by homology searching of sequence databases, both public and private. Conveniently, publicly available databases may be used, although private or commercially-available databases will be equally useful, particularly if they contain data not represented in the public databases. Primary databases are the sites of primary nucleotide or amino acid sequence data deposit and may be publicly or commercially available. Examples of publicly-available primary databases include the GenBank database (http://www.ncbi.nlm.nih.gov/), the EMBL database (http://www.ebi.ac.uk/), the DDBJ database (http://www.ddbj.nig.ac.jp/), the SWISS-PROT protein database (http://expasy.hcuge.ch/), PIR (http://pir.georgetown.edu/), TrEMBL (http://www.ebi.ac.ukc/), the TIGR databases (see http://www.tigr.org/tdb/index.html), the NRL-3D database (http://www.nbrfa.georgetown.edu), the Protein Data Base (http://www.rcsb.org/pdb), the NRDB database (ftp://ncbi.nlm.nih.gov/pub/nrdb/README), the OWL database (http://www.biochem.ucl.ac.uk/bsm/dbbrowser/OWL/) and the secondary databases PROSITE (http://expasy.hcuge.ch/sprot/prosite.html), PRINTS (http://iupab.leeds.ac.uk/bmb5dp/prints.html), Profiles (http://ulrec3.unil.ch/software/PFSCAN_form.html), Pfam (http://www.sanger.ac.uk/software/pfam), Identify (http://dna.stanford.edu/identify/) and Blocks (http://www.blocks.fhcrc.org) databases. Examples of commercially-available databases or private databases include PathoGenome (Genome Therapeutics Inc.) and PathoSeq (previously of Incyte Pharmaceuticals Inc.).

Typically, greater than 30% identity between two polypeptides (preferably, over a specified region such as the active site) is considered to be an indication of functional equivalence and thus an indication that two proteins are homologous. Preferably, proteins that are homologues have a degree of sequence identity with the EV576 protein sequence identified in Figure 2 of greater than 60%. More preferred homologues have degrees of identity of greater than 70%, 80%, 90%, 95%, 98% or 99%, respectively with the EV576 protein sequence given in Figure 2. Percentage identity, as referred to herein, is as determined using BLAST version 2.1.3 using the default parameters specified by the NCBI (the National Center for Biotechnology Information; http://www.ncbi.nlm.nih.gov/) [Blosum 62 matrix; gap open penalty=11 and gap extension penalty=1].

Homologues of the EV576 protein sequence given in Figure 2 include mutants containing amino acid substitutions, insertions or deletions from the wild type sequence, for example, of 1, 2, 3, 4, 5, 7, 10 or more amino acids, provided that such mutants retain the ability to bind C5. Mutants thus include proteins containing conservative amino acid substitutions that do not affect the function or activity of the protein in an adverse manner. This term is also intended to include natural biological variants (e.g. allelic variants or geographical variations within the species from which the EV576 proteins are derived). Mutants with improved ability to bind C5 may also be designed through the systematic or directed mutation of specific residues in the protein sequence.

Fragments of the EV576 protein and of homologues of the EV576 protein are also embraced by the term "functional equivalents" providing that such fragments retain the ability to bind C5. Fragments may include, for example, polypeptides derived from the EV576 protein sequence which are less than 150 amino acids, less than 125 amino acids, less than 100 amino acids, less than 75 amino acids, less than 50 amino acids, or even 25 amino acids or less, provided that these fragments retain the ability to bind to complement C5.

Included as such fragments are not only fragments of the *O. moubata* EV576 protein that is explicitly identified herein in Figure 2, but also fragments of homologues of this protein, as described above. Such fragments of homologues will typically possess greater than 60% identity with fragments of the EV576 protein sequence in Figure 2, although more preferred fragments of homologues will display degrees of identity of greater than 70%, 80%, 90%, 95%, 98% or 99%, respectively with fragments of the EV576 protein sequence in Figure 2. Fragments with improved may, of course, be rationally designed by the systematic mutation or fragmentation of the wild type sequence followed by appropriate activity assays. Fragments may exhibit similar or greater affinity for C5 as EV576 and may have the same or greater IC₅₀ for C5.

A functional equivalent used according to the invention may be a fusion protein, obtained, for example, by cloning a polynucleotide encoding the EV576 protein in frame to the coding sequences for a heterologous protein sequence. The term "heterologous", when used herein, is intended to designate any polypeptide other than the EV576 protein or its functional equivalent. Example of heterologous sequences, that can be comprised in the soluble fusion proteins either at N- or at C-terminus, are the following: extracellular domains of membrane-bound protein, immunoglobulin constant regions (Fc region), multimerization domains, domains of extracellular proteins, signal sequences, export sequences, or sequences allowing purification by affinity chromatography. Many of these heterologous sequences are commercially available in expression plasmids since these sequences are commonly included in the fusion proteins in order to provide additional properties without significantly impairing the specific biological activity of the protein fused to them [15]. Examples of such additional properties are a longer lasting half-life in body fluids, the extracellular localization, or an easier purification procedure as allowed by a tag such as a histidine or HA tag.

The EV576 protein and functional equivalents thereof, may be prepared in recombinant form by expression in a host cell. Such expression methods are well known to those of skill in the art and are described in detail by [16] and [17]. Recombinant forms of the EV576 protein and functional equivalents thereof are preferably unglycosylated.

The proteins and fragments of the present invention can also be prepared using conventional techniques of protein chemistry. For example, protein fragments may be prepared by chemical synthesis. Methods for the generation of fusion proteins are standard in the art and will be known to the skilled reader. For example, most general molecular biology, microbiology recombinant DNA technology and immunological techniques can be found in [16] or [18].

The subject to which the agent that binds C5 is administered in the method or use of the invention is preferably a mammal, preferably a human. The subject to which the agent that binds C5 is administered may also be suffering from a further disease with which respiratory disorders are associated, such as systemic autoimmune and connective tissue diseases such as rheumatoid arthritis, systemic lupus erythematosus, polyarteritis nodosa and systemic sclerosis.

The agent is administered in a therapeutically or prophylactically effective amount. The term "therapeutically effective amount" refers to the amount of agent needed to treat or ameliorate a targeted disease. The term "prophylactically effective amount" used herein refers to the amount of agent needed to prevent a targeted disease.

Preferably, the dose of the agent is sufficient to bind as much available C5 as possible in the subject, more preferably, all available C5. Preferably, the dose of the agent supplied is at least twice the molar dose needed to bind all available C5 in the subject. The dose of the agent supplied may be 2.5 times, 3 times or 4 times the molar dose needed to bind all available C5 in the subject. Preferably, the dose is from 0.0001 mg/kg (mass of drug compared to mass of patient) to 20 mg/kg, preferably 0.001 mg/kg to 10 mg/kg, more preferably 0.2 mg/kg to 2 mg/kg.

The frequency with which the dose needs to be administered will depend on the half-life of the agent involved. Where the agent is the EV576 protein or a functional equivalent thereof, the dose may be administered as a continuous infusion, in bolus doses or on a daily basis, twice daily basis, or every two, three, four days, five, six, seven, 10, 15 or 20 days or more.

The exact dosage and the frequency of doses may also be dependent on the patient's status at the time of administration. Factors that may be taken into consideration when determining dosage include the severity of the disease state in the patient, the general health of the patient, the age, weight, gender, diet, time and frequency of administration, drug combinations, reaction sensitivities and the patient's tolerance or response to therapy. The precise amount can be determined by routine experimentation, but may ultimately lie with the judgement of the clinician.

The agent will generally be administered as part of a pharmaceutically acceptable carrier. The term "pharmaceutically acceptable carrier", as used herein, includes genes, polypeptides, antibodies, liposomes, polysaccharides, polylactic acids, polyglycolic acids and inactive virus particles or indeed any other agent provided that the carrier does not itself induce toxicity effects or cause the production of antibodies that are harmful to the individual receiving the pharmaceutical composition. Pharmaceutically acceptable carriers may additionally contain liquids such as water, saline, glycerol, ethanol or auxiliary substances such as wetting or emulsifying agents, pH buffering substances and the like. The pharmaceutical carrier employed will thus vary depending on the route of administration. Carriers may enable the pharmaceutical compositions to be formulated into tablets, pills, dragees, capsules, liquids, gels, syrups, slurries, suspensions to aid intake by the patient. A thorough discussion of pharmaceutically acceptable carriers is available in [19].

The agent may be delivered by any known route of administration. The agent may be delivered nasally, by inhalation, for example, using a metered-dose inhaler, nebuliser, dry powder inhaler, or nasal inhaler. The agent may be delivered by a parenteral route (e.g. by injection, either subcutaneously, intraperitoneally, intravenously or intramuscularly or delivered to the interstitial space of a tissue). The compositions can also be administered into a lesion. Other modes of administration include oral and pulmonary administration, suppositories, and transdermal or transcutaneous applications, needles, and hyposprays.

The agent that binds C5 may be administered alone or as part of a treatment regimen also involving the administration of other drugs currently used in the treatment of patients with respiratory disorders. For example, the agent may be administered in combination with the infusion of a corticosteroid, immunosuppressive agent, cytotoxic agent, anti-allergic agent (e.g. antihistamine), histamine and serotonin binding molecule, chemokine and cytokine antagonist, antimicrobial, antiparasitic or with treatment such as haemodialysis or plasmapheresis. Combinations of drug treatments may have an additive or synergistic effect on treatment of the disease.

The invention thus provides -(i) an agent that binds C5, preferably the EV576 protein or a functional equivalent thereof, and (ii) one or more of a corticosteroid, immunosuppressive agent, cytotoxic agent, anti-allergic agent (e.g. antihistamine), histamine and serotonin binding molecule, chemokine and cytokine antagonist, antimicrobial, antiparasitic for use in therapy.

The invention also provides the use of (i) an agent that binds C5, preferably the EV576 protein or a functional equivalent thereof, and (ii) one or more of a corticosteroid, immunosuppressive agent, cytotoxic agent, anti-allergic agent (e.g. antihistamine), histamine and serotonin binding molecule, chemokine and cytokine antagonist, antimicrobial, antiparasitic in the manufacture of a medicament for treating respiratory disorders.

The agent that binds C5 may be administered simultaneously, sequentially or separately with the other drug(s). For example, the agent that binds C5 may be administered before or after administration of the other drug(s).

The invention thus provides the use of an agent that binds C5, preferably the EV576 protein or a functional equivalent thereof, in the manufacture of a medicament for treating a respiratory disorder in a subject, wherein said subject has been pre-treated with one or more of a coiticosteroid, immunosuppressive agent, cytotoxic agent, anti-allergic agent (e.g. antihistamine), histamine and serotonin binding molecule, chemokine and cytokine antagonist, antimicrobial, antiparasitic.

The invention also provides the use of one or more of a corticosteroid, immunosuppressive agent, cytotoxic agent, anti-allergic agent (e.g. antihistamine), histamine and serotonin binding molecule, chemokine and cytokine antagonist, antimicrobial, antiparasitic in the manufacture of a medicament for treating a respiratory disorder in a subject wherein said subject has been pre-treated with an agent that binds C5, preferably the EV576 protein or a functional equivalent thereof.

The agent that binds C5 may also be administered as part of a treatment regimen also involving the administration of other drugs currently used in the treatment of other diseases with which respiratory disorders are associated. The agent that binds C5 may be administered simultaneously, sequentially or separately with the other drug(s). For example, the agent that binds C5 may be administered before or after administration of the other drug(s).

Various aspects and embodiments of the present invention will now be described in more detail by way of example. It will be appreciated that modification of detail may be made without departing from the scope of the invention.

### Brief description of Figures:

**Figure 1****:** Schematic diagram of classical and alternative pathways of complement activation. Enzymatic components, dark grey. Anaphylatoxins enclosed in starbursts.
**Figure 2****:** Primary sequence of EV576. Signal sequence underlined. Cysteine residues in bold type. Nucleotide and amino acid number indicated at right.
**Figure 3****:** Purification of EV576 from tick salivary gland extract (SGE). A) Anion exchange chromatography. B) Classical haemolytic assay of fractions. C) Reducing SDS-PAGE. D) RP-HPLC.
**Figure 4****:** Mechanism of action of EV576. A) No effect on C3a production. B) Prevents C5a production. C) Binds directly to C5.
**Figure 5****:** Recombinant EV576. A) Recombinant EV576 (rEV576) inhibits complement as effectively as native EV576. B) Structure of EV576.
**Figure 6****:** Effect of rEV576 in acute model of asthma. A) PenH levels in mice following methacholine treatment. B) Differential cell counts from bronchoalveolar lavage fluid (BAL) after PenH induction.
**Figure 7****:** Effect of rEV576 in pulmonary immune complex model. A) rEV576 reduces vascular leak in immune complex alveolitis. B) rEV576 inhibits neutrophil recruitment in the bronchoalveolar lavage fluid (BAL) and lung in immune complex alveolitis. C) lung tissue sections demonstrate that rEV576 prevents neutrophil recruitment which is induced in lung tissue by immune complex alveolitis.

### Examples

### 1. Mechanism of action and inhibitory concentration.

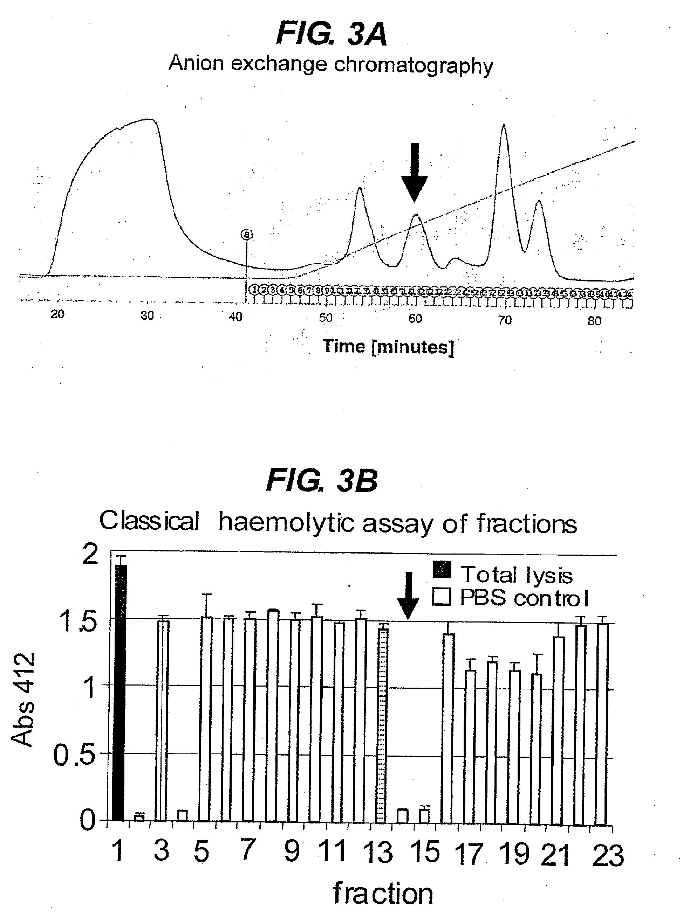

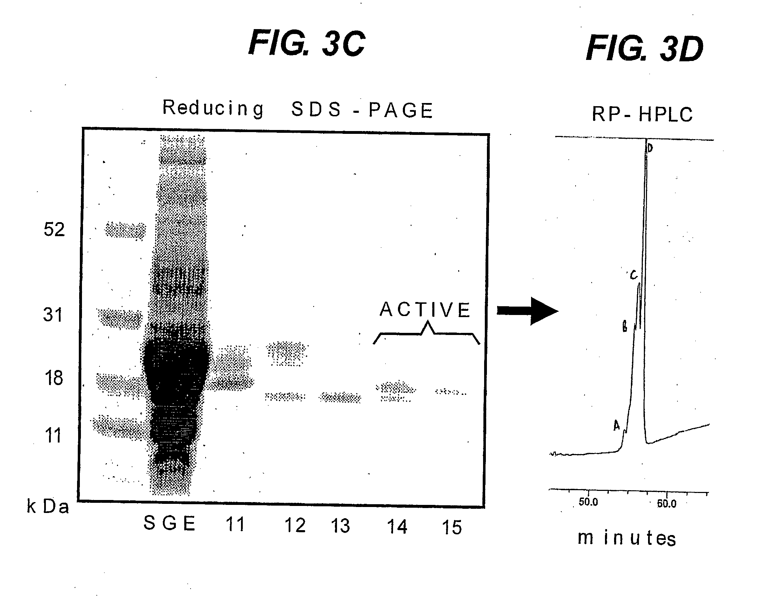

EV576 was purified from salivary gland extracts of the soft tick *Orthinodoros moubata* by SDS-PAGE and RP-HPLC of fractions of salivary gland extract found to contain complement inhibitory activity by classical haemolytic assays (Figure 3) as disclosed in [20].

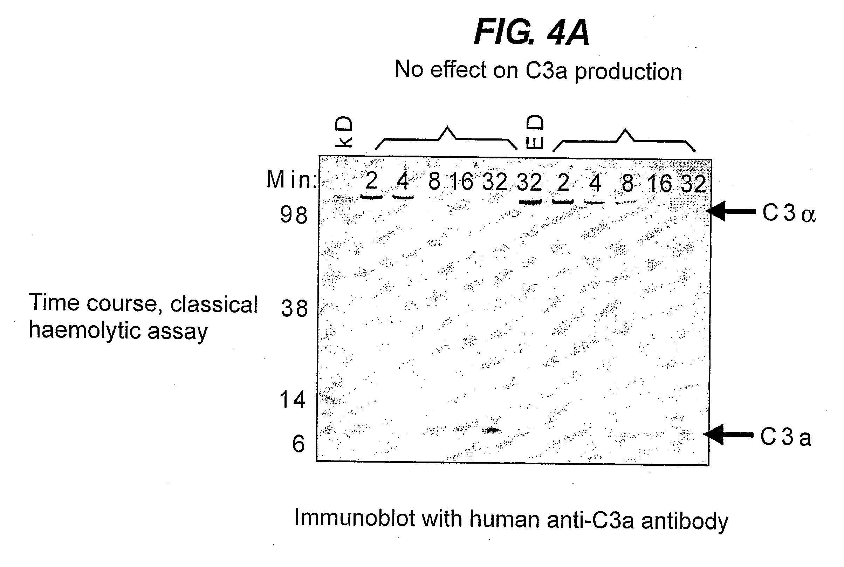

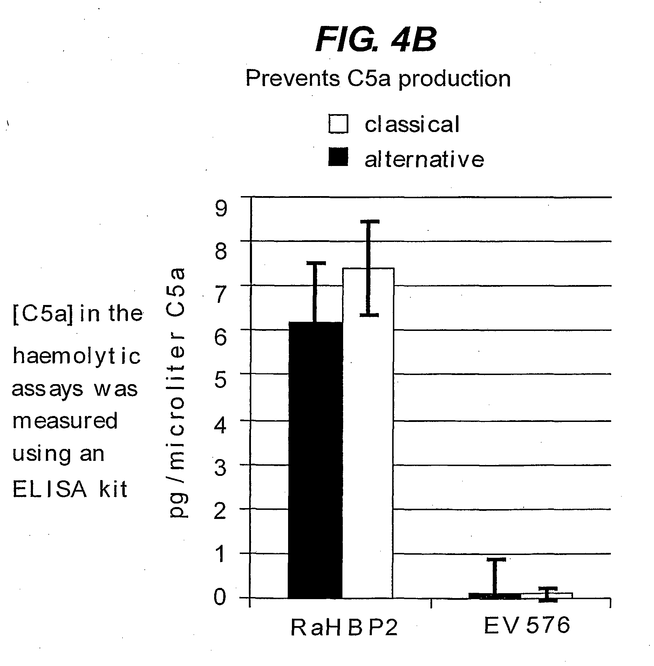

EV576 inhibits both human and guinea pig classical and alternative pathways. It has no effect on the rate of C3a production (Figure 4A) but prevents cleavage of C5a from C5 (Figure 4B).

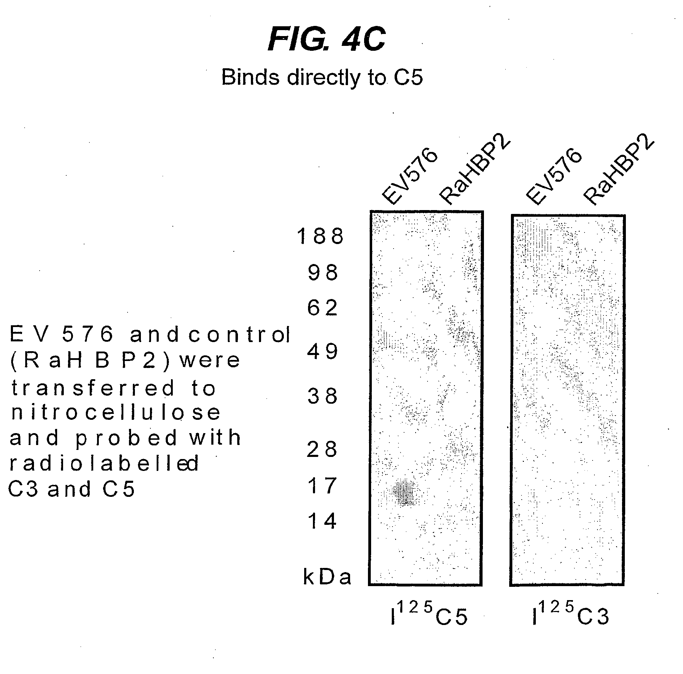

The ability of EV576 to inhibit both the classical and the alternative complement pathways is due to binding of the molecule to complement C5, the precursor of C5a and C5b - 9. EV576 binds directly to C5 (Figure 4C) with an IC₅₀ of ≈ 0.02 mg/ml. The precise binding mechanism and accessory roles (if any) played by serum factors are under investigation.

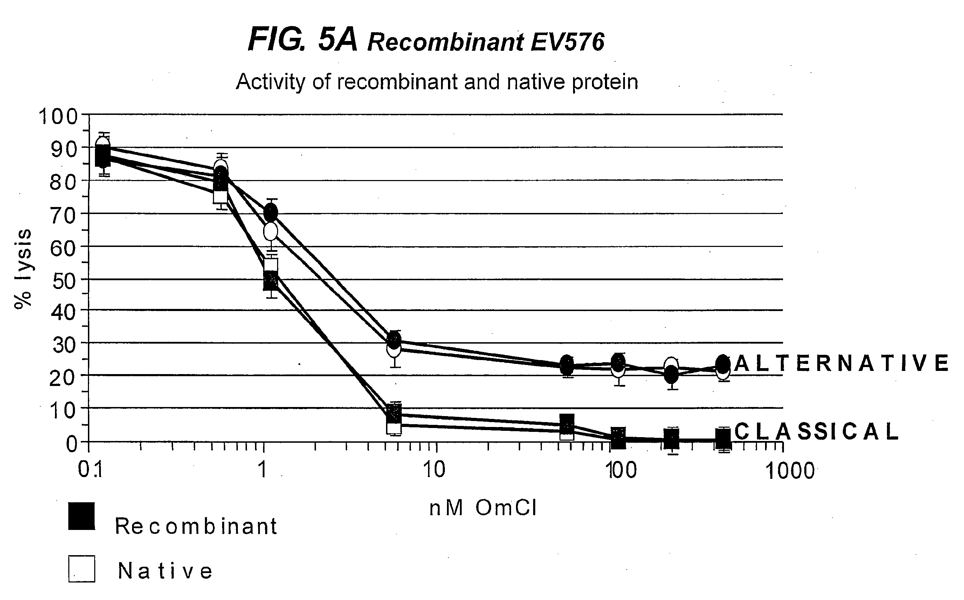

Recombinant EV576 (rEV576) with glycosylation sites removed (which otherwise are glycosylated in the yeast expression system) is as active as the native non-glycosylated protein (Figure 5A).

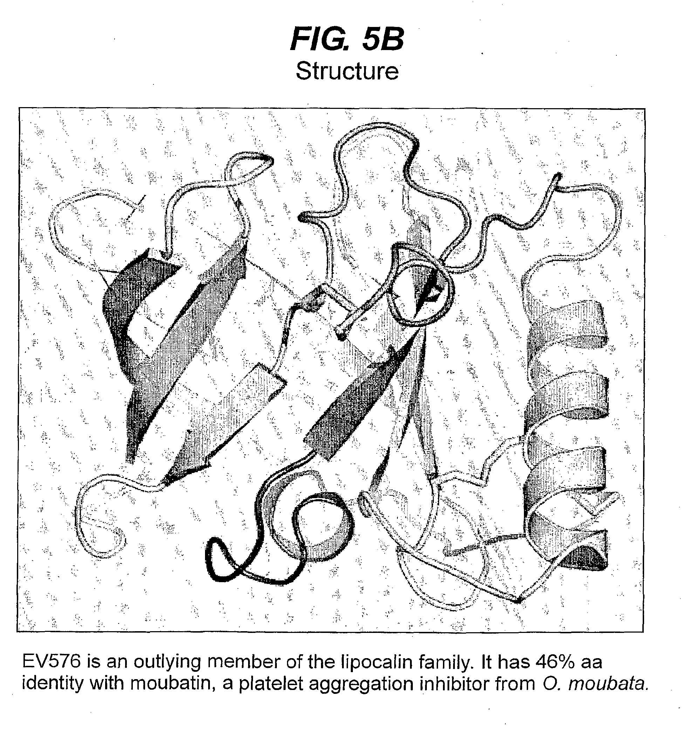

The structure of EV576 confirms that it is an outlying member of the lipocalin family (Figure 5B), having 46% identity with moubatin, a platelet aggregation inhibitor from *O. moubata.* Lipocalins are a large group of soft tick proteins the functions of which, with rare exceptions, are unknown.

### 2. Effect of EV576 on a mouse model of asthma.

The response to rEV576 at three different concentrations, in OVA-sensitised and challenged mice was compared with Budesonide treated and unsensitised/unchallenged controls.

Female Balb/c mice, aged 5-8 weeks were grouped as shown in the table below:

| **Treatment Groups (n=8)** | |
|---|---|
| A | Unsensitised/challenged/untreated |
| B | Sensitised/challenged/untreated |
| C | Sensitised/challenged/treated rEV576 at 400µg per mouse per day (20mg/kg) |
| D | Sensitised/challenged/treated rEV576 at 40µg per mouse per day (2mg/kg) |
| E | Sensitised/challenged/treated rEV576 at 4µg per mouse per day (0.2mg/kg) |
| F | Sensitised/challenged/treated budesonide 1mg in PBS |

Mice in groups B-F were sensitised to OVA by injection intraperitoneally with 10µg in 200µl OVA in alum at day 0 and day 14. All animals were then challenged by aerosol exposure to 5% OVA in DW for 20 minutes daily from day 18-23. In addition mice in groups C-F were given treatments of rEV576 by aerosolisation on days 21-24, 1 hour prior to OVA challenge. At termination (day24) all animals were exposed to increased concentrations of methacholine from 3.125 mg/ml to 50mg/ml in PBS in a methacholine challenge test. BAL fluids were collected for preparation of cytospins for analysis of infiltrating inflammatory cells.

Airway hyper-responsiveness was assessed using penH (see http://www.buxco.com/Response%20Standard.pdf). The algorithm for Penh is derived from whole body plethysmography experiments and compares the average amplitude of the early part of the expiratory phase to the average amplitude of the later part of the expiratory phase; and the peak amplitude of the expiratory phase to the peak amplitude of the inspiratory phase.

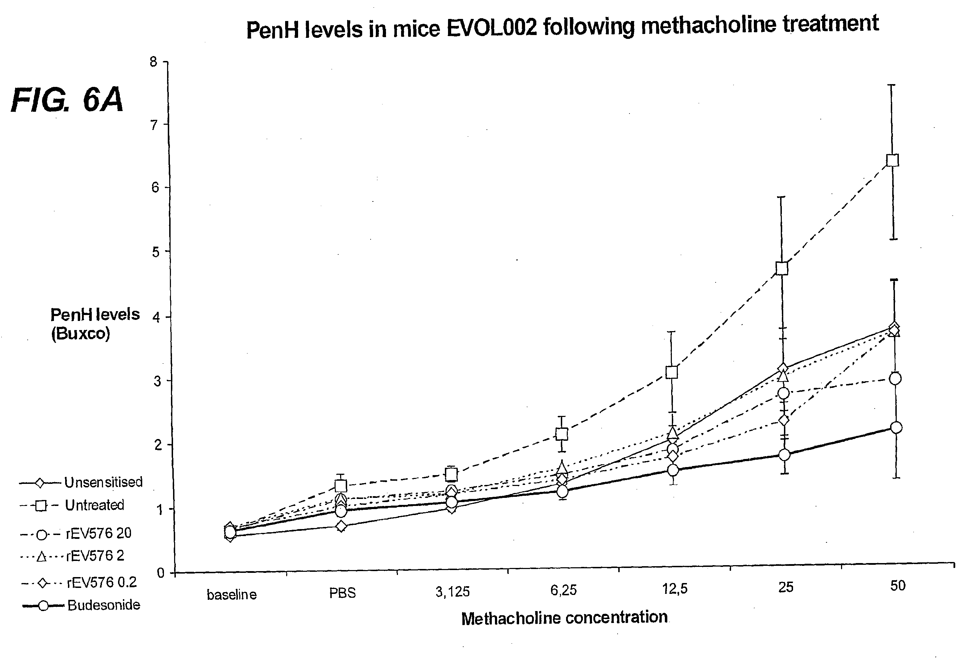

PenH responses clearly show that sensitisation with OVA/alum followed by aerosol OVA exposure resulted in airway hyper-responsiveness; penH values rose to higher levels in Group B when compared to Group A. Treatment with Budesonide (Group F) reduced the penH response markedly with mean values falling below those seen in unsensitised, but aerosol challenged mice (Group A). Similarly, treatment with rEV576 reduced penH values to background or even slightly below background levels. Even the lowest dose of rEV576 used reduced penH fully (Figure 6A).

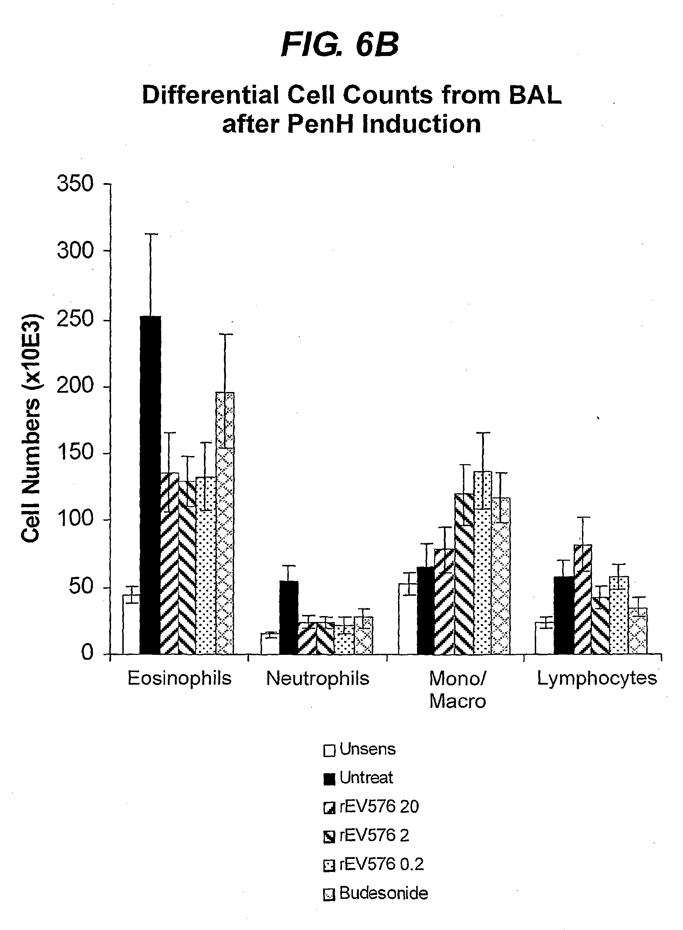

Lung cell infiltration into the bronchoalveolar lavage fluid (BAL) was also assessed. As expected, sensitisation led to a marked increase in the numbers of cells in the BAL and the dominant cell type present was eosinophils. There was also a slight increase in the number of neutrophils and lymphocytes present in the BAL of sensitised animals in comparison to those that were aerosol challenged only. Treatment with Budesonide reduced the numbers of eosinophils, neutrophils and lymphocytes in the BAL in comparison to the untreated group (B). The decrease in eosinophil numbers was less than that seen in similar experiments. Treatment with rEV576 reduced the infiltration of eosinophils and neutrophils at least as well as budesonide, with a clear difference in eosinophil numbers being evident compared to untreated mice at all doses tested. All treated were associated with a slight increase in monocyte numbers in the BAL. The numbers of monocytes was low and these differences are unlikely to be significant (no formal statistics done) (Figure 6B).

Thus rEV576 was potent in modulating acute asthma in the OVA model. It reduced airway hyperresponsiveness to background levels at all doses tested, and was able to reduce cellular infiltration into the lung. The effect on eosinophil numbers in the lung was less marked than the effect on penH, suggesting its effects were at least partly independent of any ability to control the cellular processes underlying airway reactivity.

### 3. Effect of EV576 on immune complex induced alveolitis in the lung

To investigate the effect of rEV576 on immune complex alveolitis, C57/BL6 male mice, about 8 weeks old and with a body weight of about 25 g, were injected intravenously with 300 µg Ova containing 0.3% Evans Blue (EB) together with 0, 50 or 250µg of rEV576. Immediately thereafter, 150 µg of anti-Ova antibody was applied intranasally to each mouse. 4 hours after the antibody administration, mice were killed, lungs were perfused with an isotonic solution, BAL was performed and lungs excised for MPO determination. Cells in BAL were counted and differential staining was performed using Diff-Quick [21]. The experiments were performed twice with groups of 4 animals each.

Vascular leak, neutrophil recruitment into the bronchoalveolar space (BAL), myeloperoxidase (MPO) activity in the lung and inflammation on histological sections were investigated.

As a positive control a p38 MAPK inhibitor was used, for which inhibition of LPS-induced vascular leakage had been shown [21]. The p38 MAPK inhibitor prevented the development of immune complex induced alveolitis and neutrophil recruitment completely when given intranasally and partially when given intravenously.

### rEV576 reduces vascular leak in immune complex alveolitis

The formation of immune complexes and their local deposition induces an acute inflammatory response. The vascular leakage was determined by measuring the Evans blue leaked to the bronchoalveolar space (BAL). BAL was deprived of cells by centrifugation, and the absorption was measured using an ELISA plate reader at OD 610 nm.

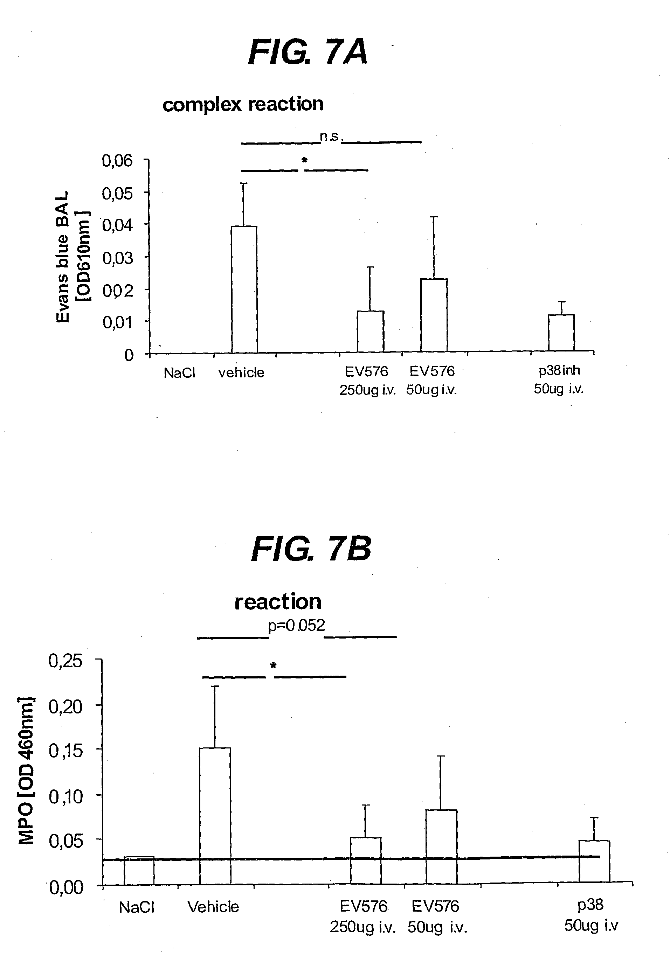

rEV576 given at 50 and 250µg by the i.v. route inhibited immune complex induced capillary leak into the BAL fluid. The MAPK inhibitor was equally effective at 50µg (Figure 7a).

### rEV576 inhibits neutrophil recruitment in the BAL and lung upon immune complex alveolitis

Immune complex induced neutrophil recruitment in the BAL was inhibited by rEV576 given at 50µg (p<0.05, n=4), and at 250µg by the i.v. route. The MAPK inhibitor had no significant effect at 50µg (data not shown).

Myeloperoxidase assay (MPO) activity in the lung tissue was evaluated as described by Lefort and colleagues [22]. In brief, the frozen lung was homogenized for 30 seconds in 1 ml ice-cold PBS with a polytron mixer. The extract was centrifuged (10000 x g, 10 min at 4o C), and the supernatant discarded. The pellet was resuspended in 1 ml PBS-HTAB-EDTA (PBS containing 0.5 % HTAB (hexadecyl-trimethyl ammonium bromide) and 1 mM EDTA), homogenized for 30 seconds and centrifuged. 100 µl of the supernatant was placed in a tube, together with 2 ml Hanks balanced salt solution (HBSS, w/Ca²⁺ and Mg²⁺), 200 µl PBS-HTAB-EDTA, 100 µl of o-dianisidine (1.25 mg/ml H2O), and 100 µl H₂O₂. After 15 min of incubation at 37° C under agitation the reaction was stopped by transfer on ice and addition of 100 µl NaN3 (1 %). The MPO activity was quantified as absorbance at 460 nm.

Neutrophil recruitment into the lung, was dose dependently inhibited by EV576 given i.v. upon immune complex reaction (50 and 250µg), as assessed by MPO measurements in the lung tissue homogenate. The MAPK inhibitor was equally effective at 50µg (Figure 7B).

### Lung histology

Immune complex induced neutrophil recruitment in the lung tissue was further investigated in lung tissue sections. After bronchoalveolar lavage, the lung was excised (4 hours after anti-Ova administration) and fixed in 4% buffered formaldehyde for standard microscopic analysis using H&E. The peribronchial infiltrate was assessed by a semi-quantitative score (0 - 3) by two independent observers.

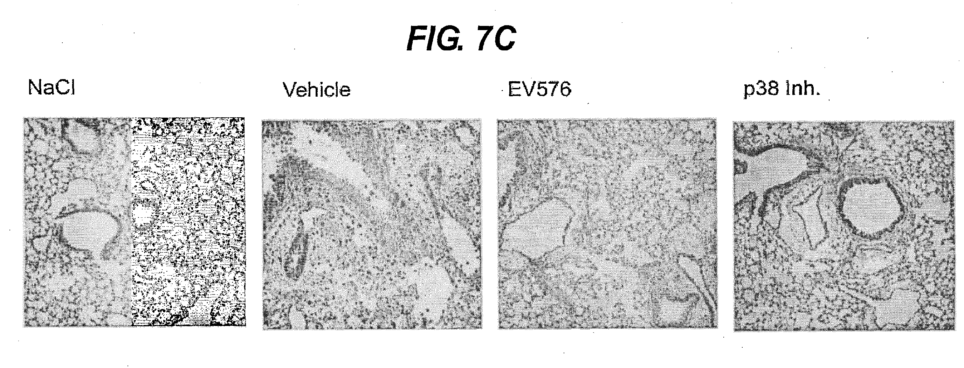

rEV576 given at 50µg by the i.v. route and the MAPK inhibitor prevented neutrophil recruitment, haemorrhage and oedema in the lung (Figure 7C).

This data demonstrates that rEV576, administered intravenously, is a potent inhibitor of capillary leak, neutrophil recruitment and haemorrhage into the lung tissue and BAL, induced by immune complex lung injury.
[1] Mastellos, D., et al., Clin Immunol, 2005. 115(3): p. 225-35
[2] Abe et al., J. Immunol. 2001. 167:4651-4660
[3] Lukacs et al., Am. J. Physiol. Lung Cell Mol. Physiol. 2001. 280:L512-L518
[4] Peng et al., The Journal of Clinical Investigation 2005. 115:6:1590-1600
[5] Guo, R.F. and P.A. Ward, Annu Rev Immunol, 2005, 23: p. 821-52
[6] Neumann, E., et al., Arthritis Rheum, 2002. 46(4): p. 934-45
[7] Williams, A.S., et al., Arthritis Rheum, 2004, 50(9): p. 3035-44
[8] Quigg, R.J., Curr Dir Autoimmun, 2004. 7: p. 165-80
[9] Papagianni, A.A., et al., Nephrol Dial Transplant, 2002, 17(1): p. 57-63
[10] He, C., et al., J Immunol, 2005. 174(9): p. 5750-7
[11] Mead, R.J., et al., J Immunol, 2002. 168(1): p. 458-65
[12] Nakashima, S., et al., J Immunol, 2002. 169(8): p. 4620-7
[13] Mizuno, M. and D.S. Cole, Expert Opin Investig Drugs, 2005. 14(7): p. 807-21
[14] Allegretti, M., et al.,. Curr Med Chem, 2005. 12(2): p. 217-36
[15] Terpe K, Appl Microbiol Biotechnol, 60: 523-33, 2003
[16] Sambrook *et al* (2000)
[17] Fernandez & Hoeffler (1998)
[18] Ausubel *et al:* (1991)
[19] Remington's Pharmaceutical Sciences; Mack Pub. Co., N.J. 1991
[20] WO2004/106369
[21] Schnyder-Candrian, S., et. al.,. J Immunol, 2005. pages 175-262*.*
[22] Lefort, J., et. al., J Immunol, 1998. pages 161-474*.*

## Claims

1. An agent that binds complement C5 for use in treating or preventing a respiratory disorder, wherein the agent that binds complement C5 isa protein comprising or consisting of amino acids 19 to 168 of the amino acid sequence of SEQ ID NO: 2 or is a homologue or fragment thereof which retains its ability to bind C5.

2. The agent for use of claim 1 wherein the agent acts to prevent the cleavage of complement C5 by C5 convertase into complement C5a and complement C5b-9.

3. The agent for use of claims 1-2 wherein the agent binds C5 with an IC₅₀ of less than 0.2 mg/ml.

4. The agent for use of any one of claims 1 to 3 wherein the agent is derived from a haematophagous arthropod.

5. The agent for use of any one of claims 1 to 4 wherein the agent that binds C5 is a protein comprising or consisting of an amino acid sequence with greater than 60% identity with the amino acid sequence of SEQ ID NO: 2.

6. The agent for use of any one of claims 1 to 5 wherein the agent that binds C5 is a protein comprising or consisting of amino acids 1 to 168 of the amino acid sequence in SEQ ID NO: 2 or is a functional equivalent of this protein.

7. The agent for use of any one of claims 1 to 5 wherein the agent is administered as a nucleic acid molecule encoding a protein as recited in any one of claims 1-6.

8. The agent for use of claim 7 wherein the nucleic acid molecule comprises or consists of bases 55 to 507 of the nucleotide sequence in SEQ ID NO: 1.

9. The agent for use of claim 8 wherein the nucleic acid molecule comprises or consists of bases 1 to 507 of the nucleotide sequence in SEQ ID NO: 1.

10. The agent for use of claims 1 to 9 wherein the subject to be treated is a mammal, preferably a human.

11. The agent for use of any one of claims 1 to 9 wherein the agent is administered in a dose sufficient to bind as much available C5 as possible in the subject, more preferably, all available C5.

12. The agent for use of any one of claims 1 to 11 wherein the agent that binds C5 is administered as part of a treatment regimen also involving the administration of a further drug for the treatment of respiratory disorders.

13. The agent for use of claim 12 wherein the further drug is selected from one or more of the group consisting of a steroid such as beclomethasone, budesonide, and fluticasone, a beta agonist such as salbutamol and terbutaline, an anticholinergic agent such as ipratropium and oxitropium, immunosuppressive agent, cytotoxic agent, anti-allergic agent (e.g. antihistamine), histamine and serotonin binding molecule, chemokine and cytokine antagonist, an antimicrobial, or an antiparasitic agent.

14. The agent for use of claim 12 or claim 13 wherein the agent that binds C5 is administered simultaneously, sequentially or separately with the further one or more drugs.

15. The agent for use of any one of claims 1 to 14 wherein the respiratory disorder is selected from the group consisting of asthma, COPD, immune complex alveolitis; asthma, rhinitis, alveolitis or diffuse fibrotic lung disease caused by exposure to systemic or inhaled drugs and chemical agents; physical lung damage; aspiration pneumonitis; lipoid pneumonia; lung damage associated with organ transplantation; cryptogenic fibrosing alveolitis; allergic granulomatosis; Wegener's granulomatosis; broncheolitis obliterans; interstitial pulmonary fibrosis; cystic fibrosis; respiratory manifestations of autoimmune and connective tissue diseases; Goodpasture's syndrome; pulmonary alveolar proteinosis; idiopathic pulmonary haemosiderosis; histiocytosis X; pulmonary infiltration with eosinophilia or lymphangioleiomyomatosis.

16. The agent for use according to claim 15 wherein:
(a) the asthma is severe and steroid resistant asthma;
(b) the immune complex alveolitis is that caused by exposure to organic dusts, moulds, airborne allergens, mineral dust or chemicals, including farmer's lung, pigeon or bird fancier's lung, barn fever, miller's lung, metalworker's lung, humidifier fever, silicosis, pneumoconiosis, asbestosis, byssinosis, berylliosis or mesothelioma.
(c) the asthma, rhinitis, alveolitis or diffuse fibrotic lung disease is caused by exposure to bleomycin, mitomycin, penicillins, sulphonamides, cephalosporins, aspirin, NSAIDs, tartrazine, ACE inhibitors, iodine containing contrast media, non-selective β blocking drugs, suxamethonium, hexamethonium, thiopentone, amiodarone, nitrofurantoin, paraquat, oxygen, cytotoxic agents, tetracyclines, phenytoin, carbamazepine, chlorpropamide, hydralazine, procainamide, isoniazid or *p*-aminosalicylic acid;
(d) the physical lung damage is crush injury, smoke and hot gas inhalation, blast injury, or radiation injury;
(e) the lung damage is associated cardiac transplantation, lung transplantation or bone marrow transplantation;
(f) the respiratory manifestations of autoimmune and connective tissue diseases include rheumatoid disease, systemic lupus erythematosus, systemic sclerosis, polyarteritis nodosa, polymyositis, dermatomyositis, Sjögren's syndrome, ankylosing spondylitis or Caplan's syndrome; or
(g) the pulmonary infiltration with eosinophilia is simple pulmonary eosinophilia, prolonged pulmonary eosinophilia, asthmatic bronchopulmonary eosinophilia, allergic bronchopulmonary aspergillosis, aspergilloma, invasive aspergillosis, tropical pulmonary eosinophilia, hypereosinohilic syndrome or parasitic infestation.

17. The agent for use according to claim 15 wherein the respiratory disorder is selected from the group consisting of asthma, and COPD (chronic obstructive pulmonary) disease).

## Patentansprüche

1. Mittel, das Komplement C5 bindet, für die Verwendung zur Behandlung oder Vorbeugung einer Atemwegsstörung, wobei das Mittel, das Komplement C5 bindet, ein Protein ist, das die Aminosäuren 19 bis 168 der Aminosäuresequenz SEQ ID NO: 2 umfasst oder daraus besteht oder ein Homologon oder Fragment davon ist, das dessen Fähigkeit zur Bindung von C5 beibehält.

2. Mittel für die Verwendung nach Anspruch 1, wobei das Mittel eine Verhinderung der Spaltung von Komplement C5 durch C5-Konvertase in Komplement C5a und Komplement C5b-9 bewirkt.

3. Mittel für die Verwendung nach den Ansprüchen 1-2, wobei das Mittel C5 mit einer IC₅₀ von weniger als 0,2 mg/ml bindet.

4. Mittel für die Verwendung nach einem der Ansprüche 1 bis 3, wobei das Mittel von einem hämatophagen Arthropoden stammt.

5. Mittel für die Verwendung nach einem der Ansprüche 1 bis 4, wobei das Mittel, das C5 bindet, ein Protein ist, das eine Aminosäuresequenz mit mehr als 60% Identität mit der Aminosäuresequenz SEQ ID NO: 2 umfasst oder daraus besteht.

6. Mittel für die Verwendung nach einem der Ansprüche 1 bis 5, wobei das Mittel, das C5 bindet, ein Protein ist, das die Aminosäuren 1 bis 168 der Aminosäuresequenz in SEQ ID NO: 2 umfasst oder daraus besteht oder ein funktionelles Äquivalent dieses Proteins ist.

7. Mittel für die Verwendung nach einem der Ansprüche 1 bis 5, wobei das Mittel als Nukleinsäuremolekül verabreicht wird, das ein Protein nach einem der Ansprüche 1-6 codiert.

8. Mittel für die Verwendung nach 7, wobei das Nukleinsäuremolekül die Basen 55 bis 507 der Nukleotidsequenz in SEQ ID NO: 1 umfasst oder daraus besteht.

9. Mittel für die Verwendung nach 8, wobei das Nukleinsäuremolekül die Basen 1 bis 507 der Nukleotidsequenz in SEQ ID NO: 1 umfasst oder daraus besteht.

10. Mittel für die Verwendung nach einem der Ansprüche 1 bis 9, wobei das zu behandelnde Individuum ein Säuger, vorzugsweise ein Mensch, ist.

11. Mittel für die Verwendung nach einem der Ansprüche 1 bis 9, wobei das Mittel in einer Dosis verabreicht wird, die ausreicht, um so viel verfügbares C5 wie möglich in dem Individuum, stärker bevorzugt sämtliches verfügbares C5, zu binden.

12. Mittel für die Verwendung nach einem der Ansprüche 1 bis 11, wobei das Mittel, das C5 bindet, als Teil eines Behandlungsschemas verabreicht wird, das auch die Verabreichung eines weiteren Arzneistoffs zur Behandlung von Atemwegsstörungen beinhaltet.

13. Mittel für die Verwendung nach Anspruch 12, wobei der weitere Arzneistoff aus einem oder mehreren aus der Gruppe ausgewählt wird, bestehend aus einem Steroid, wie Beclomethason, Budesonid und Fluticason, einem Beta-Agonisten, wie Salbutamol und Terbutalin, einem anticholinergen Mittel, wie Ipratropiumbromid und Oxitropium, immunsuppressiven Mittel, zytotoxischen Mittel, Antiallergikum (z. B. Antihistamin), Histamin- und Serotonin-bindenden Molekül, Chemokin- und Cytokin-Antagonisten, einem Mikrobizid oder einem Antiparasitenmittel.

14. Mittel für die Verwendung nach Anspruch 12 oder Anspruch 13, wobei das Mittel, das C5 bindet, gleichzeitig, nacheinander oder getrennt von dem bzw. den weiteren Arzneistoff(en) verabreicht wird.

15. Mittel für die Verwendung nach einem der Ansprüche 1 bis 14, wobei die Atemwegsstörung aus der Gruppe ausgewählt wird, bestehend aus Asthma, COPD, Immunkomplex-Alveolitis; Asthma, Rhinitis, Alveolitis oder diffuser fibrotischer Lungenerkrankung, die durch Exposition gegenüber systemischen oder inhalierten Arzneistoffen und chemischen Mitteln verursacht wird; physischer Lungenschädigung; Aspirationspneumonie; Lipoidpneumonie; Lungenschädigung in Verbindung mit Organtransplantation; kryptogener fibrosierender Alveolitis; allergischer Granulomatose; Wegener-Granulomatose; Bronchiolitis obliterans; interstitieller Lungenfibrose; zystischer Fibrose; Atemwegsmanifestationen von Autoimmun- und Bindegewebserkrankungen; Goodpasture-Syndrom; pulmonaler Alveolarproteinose; idiopathischer pulmonaler Hämosiderose; Histiozytose X; pulmonaler Infiltration mit Eosinophilie oder Lymphangioleiomyomatose.

16. Mittel für die Verwendung nach Anspruch 15, wobei:
(a) es sich bei dem Asthma um schweres und steroidresistentes Asthma handelt,
(b) es sich bei der Immunkomplex-Alveolitis um diejenige handelt, die durch Exposition gegenüber organischen Stäuben, Schimmelpilzen, luftübertragenen Allergenen, Mineralstaub oder Chemikalien hervorgerufen wird, einschließlich Farmerlunge, Tauben- oder Vogelhalterlunge, "Barn Fever", Mehlstauballergie, Metallarbeiterlunge, Luftbefeuchterfieber, Silikose, Pneumokoniose, Asbestose, Byssinose, Berylliose oder Mesotheliom;
(c) Asthma, Rhinitis, Alveolitis oder diffuse fibrotische Lungenerkrankung durch Exposition gegenüber Bleomycin, Mitomycin, Penicillinen, Sulfonamiden, Cephalosporinen, Aspirin, NSAIDs, Tartrazin, ACE-Hemmern, iodhaltigen Kontrastmedien, nicht-selektiven β-Blockern, Suxamethonium, Hexamethonium, Thiopenton, Amiodaron, Nitrofurantoin, Paraquat, Sauerstoff, zytotoxischen Mitteln, Tetrazyklinen, Phenytoin, Carbamazepin, Chlorpropamid, Hydralazin, Procainamid, Isoniazid oder p-Aminosalicylsäure verursacht wird;
(d) die physische Lungenschädigung eine Quetschverletzung, Inhalation von Rauch und heißem Gas, Explosionsverletzung oder Strahlungsverletzung ist;
(e) die Lungenschädigung mit Herztransplantation, Lungentransplantation oder Knochenmarktransplantation in Zusammenhang steht;
(f) die Atemwegsmanifestationen von Autoimmun- und Bindegewebserkrankungen rheumatoide Erkrankung, systemischen Lupus erythematodes, systemische Sklerose, Polyarteriitis nodosa, Polymyositis, Dermatomyositis, Sjögren-Syndrom, Spondylitis ankylosans oder Caplan-Syndrom beinhalten oder
(g) es sich bei der Lungeninfiltration mit Eosinophilie um einfache pulmonale Eosinophilie, länger andauernde pulmonale Eosinophilie, asthmatische bronchopulmonale Eosinophilie, allergische bronchopulmonale Aspergillose, Aspergillom, invasive Aspergillose, tropische pulmonale Eosinophilie, Hypereosinohilie-Syndrom oder Parasitenbefall handelt.

17. Mittel für die Verwendung nach Anspruch 15, wobei die Atemwegsstörung aus der Gruppe ausgewählt wird, die aus Asthma und COPD (chronisch obstruktive Lungenerkrankung) besteht.

## Revendications

1. Agent qui se lie au complément C5 pour utilisation dans le traitement ou la prévention d'un trouble respiratoire, l'agent qui se lie au complément C5 étant une protéine comprenant ou étant constituée des acides aminés 19 à 168 de la séquence d'acides aminés de SEQ ID NO: 2 ou étant un homologue ou un fragment de celui-ci qui conserve sa capacité à se lier à C5.

2. Agent pour utilisation de la revendication 1, l'agent agissant de façon à prévenir le clivage du complément C5 par la C5 convertase en complément C5a et en complément C5b-9.

3. Agent pour utilisation pour utilisation des revendications 1 à 2, l'agent se liant à C5 avec une CI₅₀ inférieure à 0,2 mg/ml.

4. Agent pour utilisation de l'une quelconque des revendications 1 à 3, l'agent étant dérivé d'un arthropode hématophage.

5. Agent pour utilisation de l'une quelconque des revendications 1 à 4, l'agent qui se lie à C5 étant une protéine comprenant ou étant constituée d'une séquence d'acides aminés ayant plus de 60 % d'identité à la séquence d'acides aminés de SEQ ID NO: 2.

6. Agent pour utilisation de l'une quelconque des revendications 1 à 5, l'agent qui se lie à C5 étant une protéine comprenant ou étant constituée des acides aminés 1 à 168 de la séquence d'acides aminés dans SEQ ID NO: 2 ou étant un équivalent fonctionnel de cette protéine.

7. Agent pour utilisation de l'une quelconque des revendications 1 à 5, l'agent étant administré sous la forme d'une molécule d'acide nucléique codant pour une protéine telle que décrite dans l'une quelconque des revendications 1 à 6.

8. Agent pour utilisation de la revendication 7, la molécule d'acide nucléique comprenant ou étant constituée des bases 55 à 507 de la séquence nucléotidique dans SEQ ID NO: 1.

9. Agent pour utilisation de la revendication 8, la molécule d'acide nucléique comprenant ou étant constitué des bases 1 à 507 de la séquence nucléotidique dans SEQ ID NO: 1.

10. Agent pour utilisation des revendications 1 à 9, le sujet à traiter étant un mammifère, de préférence un humain.

11. Agent pour utilisation de l'une quelconque des revendications 1 à 9, l'agent étant administré à une dose suffisante pour se lier à autant de C5 disponible que possible chez le sujet, plus préférablement, l'intégralité du C5 disponible.

12. Agent pour utilisation de l'une quelconque des revendications 1 à 11, l'agent qui se lie à C5 étant administré en tant que partie d'un régime de traitement mettant également en oeuvre l'administration d'un autre médicament pour le traitement de troubles respiratoires.

13. Agent pour utilisation de la revendication 12, le médicament supplémentaire étant choisi parmi un ou plusieurs du groupe constitué d'un stéroïde tel que la béclométhasone, le budésonide, et la fluticasone, un bêta-agoniste tel que le salbutamol et la terbutaline, un agent anticholinergique tel que l'ipratropium et l'oxitropium, un agent immunosuppresseur, un agent cytotoxique, un agent antiallergique (par exemple, un antihistaminique), une molécule de liaison de l'histamine et de la sérotonine, un antagoniste de chimiokine et de cytokine, un antimicrobien, ou un agent antiparasitaire.

14. Agent pour utilisation de la revendication 12 ou la revendication 13, l'agent qui se lie à C5 étant administré simultanément, séquentiellement ou séparément avec les un ou plusieurs autres médicaments.

15. Agent pour utilisation de l'une quelconque des revendications 1 à 14, le trouble respiratoire étant choisi dans le groupe constitué de l'asthme, la BPCO, l'alvéolite à complexes immuns ; l'asthme, la rhinite, l'alvéolite ou une maladie pulmonaire fibrotique diffuse causée par l'exposition à des médicaments systémiques ou inhalés et à des agents chimiques ; une atteinte physique des poumons ; une pneumonie d'aspiration ; une pneumopathie lipidique ; une atteinte pulmonaire associés à une greffe d'organe ; l'alvéolite fibrosante cryptogénique ; la granulomatose allergique ; la granulomatose de Wegener ; la bronchiolite oblitérante ; la fibrose pulmonaire interstitielle ; la fibrose kystique ; des manifestations respiratoires de maladies auto-immunes et du tissu conjonctif ; le syndrome de Goodpasture ; la protéinose alvéolaire pulmonaire ; l'hémosidérose pulmonaire idiopathique ; l'histiocytose X ; une infiltration pulmonaire avec éosinophilie ou lymphangioléïomyomatose.

16. Agent pour utilisation selon la revendication 15, où :
(a) l'asthme est un asthme sévère et résistant aux stéroïdes ;
(b) l'alvéolite à complexes immuns est celle causée par une exposition à des poussières organiques, des moisissures, des allergènes en suspension dans l'air, une poussière minérale ou des substances chimiques, comprenant le poumon de fermier, la maladie des éleveurs d'oiseaux, la fièvre des granges, le poumon de meunier, le poumon de métallurgiste, la fièvre déshumidificateur, la silicose, la pneumoconiose, l'asbestose, la byssinose, la berrylliose ou un mésothéliome,
(c) l'asthme, la rhinite, l'alvéolite au la maladie pulmonaire fibrotique diffuse est causée par une exposition à la bléomycine, la mitomycine, les pénicillines, les sulfonamides, les céphalosporines, l'aspirine, les NSAID, la tartrazine, les inhibiteurs de ACE, un agent de contraste contenant de l'iode, des médicaments β-bloquants non sélectifs, le suxaméthonium, l'hexaméthonium, la thiopentone, l'amiodarone, la nitrofurantoïne, le paraquat, l'oxygène, des agents cytotoxiques, des tétracyclines, la phénytoïne, la carbamazépine, le chlorpropamide, l'hydralazine, le procaïnamide, l'isoniazid ou l'acide *p*-aminosalicylique ;
(d) l'atteinte physique des poumons est une lésion par écrasement, l'inhalation de fumée et de gaz chaud, une lésion par explosion, ou une lésion par rayonnement ;
(e) l'atteinte pulmonaire est associée à une greffe cardiaque, une greffe de poumons ou une greffe de moelle osseuse ;
(f) les manifestations respiratoires de maladies auto-immunes et du tissu conjonctif comprennent la polyarthrite rhumatoïde, le lupus érythémateux disséminé, la sclérose en plaques, la polyartérite noueuse, la polymyosite, la dermatomyosite, le syndrome de Sjögren, la spondylarthrite ankylosante ou le syndrome de Caplan ; ou
(g) la filtration pulmonaire avec éosinophilie est l'éosinophilie pulmonaire simple, l'éosinophilie pulmonaire prolongée, l'éosinophilie bronchopulmonaire asthmatique, l'aspergillose broncho-pulmonaire allergique, l'aspergillome, l'aspergillose invasive, l'éosinophilie pulmonaire tropicale, le syndrome éosinophilique ou une infestation parasitaire.

17. Agent pour utilisation selon la revendication 15, le trouble respiratoire étant choisi dans le groupe constitué de l'asthme, et la bronchopneumopathie chronique obstructive (BPCO).


### SEQUENCE LISTING

<110> VARLEIGH LIMITED
<120> METHOD OF TREATING RESPIRATORY DISORDERS
<130> P044811EP
<140> PCT/GB2007/003404
   <141> 2007-09-10
<150> GB0617735.6
   <151> 2006-09-08
<150> US60/860730
   <151> 2006-11-22
<160> 2
<170> SeqWin99, version 1.02
<210> 1
   <211> 507
   <212> DNA
   <213> Ornithodoros moubata
<400> 1
   atgctggttt tggtgaccct gattttctcc ttttctgcga acatcgcata tgctgacagc   60
   gaaagcgact gcactggaag cgaacctgtt gacgccttcc aagctttcag tgagggcaaa  120
   gaggcatatg tcctggtgag gtccacggat cccaaagcga gggactgctt gaaaggagaa  180
   ccagccggag aaaagcagga caacacgttg ccggtgatga tgacgtttaa gaatggcaca  240
   gactgggctt caaccgattg gacgtttact ttggacggcg caaaggtaac ggcaaccctt  300
   ggtaacctaa cccaaaatag ggaagtggtc tacgactcgc aaagtcatca ctgccacgtt  360
   gacaaggtcg agaaggaagt tccagattat gagatgtgga tgctcgatgc gggagggctt  420
   gaagtggaag tcgagtgctg ccgtcaaaag cttgaagagt tggcgtctgg caggaaccaa  480
   atgtatcccc atctcaagga ctgctag                                       507
<210> 2
   <211> 168
   <212> PRT
   <213> Ornithodoros moubata
<400> 2
   Met Leu Val Leu Val Thr Leu Ile Phe Ser Phe Ser Ala Asn Ile Ala
   1               5                   10                  15
   Tyr Ala Asp Ser Glu Ser Asp Cys Thr Gly Ser Glu Pro Val Asp Ala
               20                  25                  30
   Phe Gln Ala Phe Ser Glu Gly Lys Glu Ala Tyr Val Leu Val Arg Ser
           35                  40                  45
   Thr Asp Pro Lys Ala Arg Asp Cys Leu Lys Gly Glu Pro Ala Gly Glu
       50                  55                  60
   Lys Gln Asp Asn Thr Leu Pro Val Met Met Thr Phe Lys Asn Gly Thr
   65                  70                  75                  80
   Asp Trp Ala Ser Thr Asp Trp Thr Phe Thr Leu Asp Gly Ala Lys Val
                   85                  90                  95
   Thr Ala Thr Leu Gly Asn Leu Thr Gln Asn Arg Glu Val Val Tyr Asp
               100                 105                 110
   Ser Gln Ser His His Cys His Val Asp Lys Val Glu Lys Glu Val Pro
           115                 120                 125
   Asp Tyr Glu Met Trp Met Leu Asp Ala Gly Gly Leu Glu Val Glu Val
       130                 135                 140
   Glu Cys Cys Arg Gln Lys Leu Glu Glu Leu Ala Ser Gly Arg Asn Gln
   145                 150                 155                 160
   Met Tyr Pro His Leu Lys Asp Cys
                   165

### SEQUENCE LISTING

<110> VARLEIGH LIMITED
<120> METHOD OF TREATING RESPIRATORY DISORDERS
<130> P044811EP
<140> PCT/GB2007/003404
   <141> 2007-09-10
<150> GB0617735.6
   <151> 2006-09-08
<150> US60/860730
   <151> 2006-11-22
<160> 2
<170> SeqWin99, version 1.02
<210> 1
   <211> 507
   <212> DNA
   <213> Ornithodoros moubata
<400> 1
   atgctggttt tggtgaccct gattttctcc ttttctgcga acatcgcata tgctgacagc   60
   gaaagcgact gcactggaag cgaacctgtt gacgccttcc aagctttcag tgagggcaaa  120
   gaggcatatg tcctggtgag gtccacggat cccaaagcga gggactgctt gaaaggagaa  180
   ccagccggag aaaagcagga caacacgttg ccggtgatga tgacgtttaa gaatggcaca  240
   gactgggctt caaccgattg gacgtttact ttggacggcg caaaggtaac ggcaaccctt  300
   ggtaacctaa cccaaaatag ggaagtggtc tacgactcgc aaagtcatca ctgccacgtt  360
   gacaaggtcg agaaggaagt tccagattat gagatgtgga tgctcgatgc gggagggctt  420
   gaagtggaag tcgagtgctg ccgtcaaaag cttgaagagt tggcgtctgg caggaaccaa  480
   atgtatcccc atctcaagga ctgctag                                      507
<210> 2
   <211> 168
   <212> PRT
   <213> Ornithodoros moubata
<400> 2
   Met Leu Val Leu Val Thr Leu Ile Phe Ser Phe Ser Ala Asn Ile Ala
   1               5                   10                  15
   Tyr Ala Asp Ser Glu Ser Asp Cys Thr Gly Ser Glu Pro Val Asp Ala
               20                  25                  30
   Phe Gln Ala Phe Ser Glu Gly Lys Glu Ala Tyr Val Leu Val Arg Ser
           35                  40                  45
   Thr Asp Pro Lys Ala Arg Asp Cys Leu Lys Gly Glu Pro Ala Gly Glu
       50                  55                  60
   Lys Gln Asp Asn Thr Leu Pro Val Met Met Thr Phe Lys Asn Gly Thr
   65                  70                  75                  80
   Asp Trp Ala Ser Thr Asp Trp Thr Phe Thr Leu Asp Gly Ala Lys Val
                   85                  90                  95
   Thr Ala Thr Leu Gly Asn Leu Thr Gln Asn Arg Glu Val Val Tyr Asp
               100                 105                 110
   Ser Gln Ser His His Cys His Val Asp Lys Val Glu Lys Glu Val Pro
           115                 120                 125
   Asp Tyr Glu Met Trp Met Leu Asp Ala Gly Gly Leu Glu Val Glu Val
       130                 135                 140
   Glu Cys Cys Arg Gln Lys Leu Glu Glu Leu Ala Ser Gly Arg Asn Gln
   145                 150                 155                 160
   Met Tyr Pro His Leu Lys Asp Cys
                   165